# EUROPÄISCHE PATENTSCHRIFT

(11) **EP 0 823 928 B1**
(45) Veröffentlichungstag und Bekanntmachung des Hinweises auf die Patenterteilung: **07.03.2001**
(21) Anmeldenummer: 96914970.7
(22) Anmeldetag: 22.04.1996
(51) Int. Cl.: C09C 1/00, C09D 7/12, C09D 11/00, C08K 3/00, C03C 4/02, C04B 33/14, A61K 7/00

(54) **GONIOCHROMATISCHE GLANZPIGMENTE MIT METALLSULFIDHALTIGER BESCHICHTUNG**
GONIOCHROMATIC BRIGHT PIGMENTS WITH A METAL SULPHIDE-CONTAINING COATING
PIGMENTS BRILLANTS GONIOCHROMATIQUES POURVUS D'UN RECOUVREMENT CONTENANT DES SULFURES METALLIQUES

(30) Priorität: 02.05.1995 DE 19515988
(43) Veröffentlichungstag der Anmeldung: 18.02.1998
(73) Patentinhaber: BASF AKTIENGESELLSCHAFT, 67056 Ludwigshafen (DE)
(72) Erfinder: SCHMID, Raimund, D-67435 Neustadt (DE); MRONGA, Norbert, D-69221 Dossenheim (DE); GONZALEZ GOMEZ, Juan, Antonio, D-67063 Ludwigshafen (DE)
(86) Internationale Anmeldenummer: EP9601676
(87) Internationale Veröffentlichungsnummer: WO9634917

(56) Entgegenhaltungen:
- EP-A- 0 571 836
- EP-A- 0 579 091
- EP-A- 0 668 329

## Beschreibung

Die vorliegende Erfindung betrifft ein neues Verfahren zur Herstellung von goniochromatischen Glanzpigmenten auf der Basis von mehrfach beschichteten plättchenförmigen metallischen Substraten, die mindestens ein Schichtpaket aus
A) einer im wesentlichen aus Siliciumoxid, Siliciumoxidhydrat, Aluminiumoxid und/oder Aluminiumoxidhydrat bestehenden Schicht und
B) einer nicht selektiv absorbierenden, für sichtbares Licht zumindest teilweise durchlässigen, metallsulfidhaltigen Schicht
sowie gewünschtenfalls zusätzlich
C) eine äußere, im wesentlichen aus farblosem oder selektiv absorbierendem Metalloxid bestehende Schicht
aufweisen.

Glanz- oder Effektpigmente werden in vielen Bereichen der Technik eingesetzt, beispielsweise in Automobillacken, in der dekorativen Beschichtung, der Kunststoffeinfärbung, in Anstrich-, Druck-, insbesondere Sicherheitsdruckfarben sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Aufgrund ihrer nicht kopierbaren optischen Effekte gewinnen diese Pigmente zunehmende Bedeutung für die Herstellung von fälschungssicheren Wertschriften, wie Geldscheinen, Schecks, Scheckkarten, Kreditkarten, Steuermarken, Briefmarken, Bahn- und Flugtickets, Telefonkarten, Lotterielosen, Geschenkzertifikaten, Ausweisen und Identifikationskarten.

Kennzeichnungen, die mit den Effektpigmenten angefertigt wurden, und das Fehlen dieser Kennzeichnungen oder ihre Veränderung, beispielsweise in einer Farbkopie (Verschwinden von Farbflops und Glanzeffekten), sind ohne Hilfsmittel mit bloßem Auge sicher erkennbar und ermöglichen so eine leichte Unterscheidung des Originals von der Kopie.

Glanzpigmente auf Basis metallischer Substrate sind aufgrund ihres guten Deckvermögens auch für Automobillackierungen von besonderem Interesse.

In der EP-A-579 091 werden Glanzpigmente auf der Basis von Aluminiumplättchen sowie Glimmerplättchen beschrieben, bei denen eine metallsulfidhaltige Schicht direkt oder nach vorangehender Beschichtung mit hochbrechendem Titandioxid auf die Substratplättchen aufgebracht wird. Bei Anwesenheit der TiO₂-Schicht werden Glanzpigmente erhalten, die im Glanzwinkel intensive Interferenzfarben zeigen, die jedoch bei steileren Betrachtungswinkeln zunehmend schwächer werden und schließlich in Schwarz übergehen. Goniochromatische Glanzpigmente, die einen winkelabhängigen Farbwechsel zwischen zwei Interferenzfarben ("two-tone Effekt") aufweisen, werden nicht beschrieben.

Die nicht vorveröffentlichte DE-A-44 05 492 betrifft goniochromatische Glanzpigmente auf die Basis von mit SiO₂- und Metallschichten belegten Aluminiumplättchen.

Der Erfindung lag die Aufgabe zugrunde, weitere goniochromatische Glanzpigmente mit vorteilhaften Anwendungseigenschaften bereitzustellen.

Demgemäß wurde ein Verfahren zur Herstellung von goniochromatischen Glanzpigmenten auf der Basis von mehrfach beschichteten plättchenförmigen metallischen Substraten, die mindestens ein Schichtpaket aus
A) einer im wesentlichen aus Siliciumoxid, Siliciumoxidhydrat, Aluminiumoxid und/oder Aluminiumoxidhydrat bestehenden Schicht und
B) einer nichtselektiv absorbierenden, für sichtbares Licht zumindest teilweise durchlässigen, metallsulfidhaltigen Schicht
sowie gewünschtenfalls zusätzlich
C) eine äußere, im wesentlichen aus farblosem oder selektiv absorbierendem Metalloxid bestehende Schicht
aufweisen, gefunden, welches dadurch gekennzeichnet ist, daß man
die Schichten (A) durch Gasphasenzersetzung flüchtiger Siliciumorganyle, die mindestens einen Alkanoyloxyrest enthalten, mit Wasserdampf und/oder Sauerstoff oder naßchemisch durch hydrolytische Zersetzung organischer Silicium- oder Aluminiumverbindungen, bei denen die organischen Reste über Sauerstoffatome an das Metall gebunden sind, in Gegenwart eines organischen Lösungsmittels, in welchem die Metallverbindungen löslich sind, und anschließende Trocknung,
die Schichten (B) durch Gasphasenzersetzung flüchtiger Metallverbindungen in Gegenwart eines Inertgases oder von Sauerstoff und/oder Wasserdampf und anschließende Umsetzung der gebildeten Metall- oder Metalloxidschicht mit einer flüchtigen schwefelhaltigen Verbindung oder Schwefeldampf oder direkt durch Gasphasenzersetzung der Metallverbindungen in Gegenwart einer flüchtigen schwefelhaltigen Verbindung oder Schwefeldampf,
die Schicht (C) durch Gasphasenzersetzung flüchtiger Metallverbindungen in Gegenwart von Sauerstoff und/oder Wasserdampf
auf die metallischen Substratplättchen aufbringt.

Für die erfindungsgemäß herzustellenden Glanzpigmente sind als Substrat alle für Metalleffektpigmente bekannten Metalle und Legierungen in Plättchenform geeignet. Z.B. kommen neben Stahl, Kupfer und seinen Legierungen wie Messing und Bronzen vor allem Aluminium und seine Legierungen wie Aluminiumbronze in Betracht.

Bevorzugt sind Aluminiumflakes, die in einfacher Weise durch Herausstanzen aus Aluminiumfolie oder nach gängigen Verdüsungs- und Mahltechniken herzustellen sind.

So sind beispielsweise Aluminiumplättchen geeignet, die nach dem sogenannten Hall-Verfahren in Testbenzin durch Naßmahlung hergestellt werden. Ausgangsmaterial ist ein atomisierter, spratziger Aluminiumgrieß, welcher in Kugelmühlen in Testbenzin und in Gegenwart eines Schmiermittels zu plättchenförmigen Teilchen verformt bzw. zerkleinert und anschließend klassiert wird.

Es können handelsübliche Produkte eingesetzt werden. Jedoch sollte die Oberfläche der Aluminiumteilchen weitgehend frei von Fetten oder anderen Belegmitteln sein. Diese Substanzen können zum Teil durch Lösungsmittelbehandlung oder besser, wie in der DE-A-42 23 384 beschrieben, durch oxidative Behandlung entfernt werden.

Weiterhin können die metallischen Substratteilchen passiviert sein, d.h. insbesondere gegenüber Wasser stabilisierende Beschichtungen aufweisen, wie sie z.B. aus der DE-A-42 36 332 und der nicht vorveröffentlichten DE-A-44 14 079 bekannt sind.

Als passivierende Beschichtungen sollen dabei auch Metalloxidschichten verstanden werden. Beispiele für weitere geeignete Substrate sind daher eisenoxidbeschichtete Metallpigmente (z.B. EP-A-33 457) mit (schwacher) goldener bis roter Eigenfarbe und zart pastellfarbene titandioxidbeschichtete Metallpigmente (z.B. EP-A-338 428). Die Metalloxidschicht sollte jedoch nicht zu dick sein, damit die Substratteilchen ihre "metallische Koloristik" behalten.

Schließlich sind auch magnetisierbare Aluminiumplättchen als Substratmaterial geeignet, die eine ferromagnetische, Eisen, Cobalt, Nickel, Magnetit oder γ-Fe₂O₃ enthaltende Beschichtung aufweisen (DE-A-43 13 541 und die nicht vorveröffentlichten DE-A-43 40 141 und 44 19 173) und die Herstellung magnetisierbarer goniochromatischer Glanzpigmente ermöglichen.

Die Größe der Substratteilchen ist an sich nicht kritisch und kann auf den jeweiligen Anwendungszweck abgestimmt werden. In der Regel haben die Teilchen mittlere größte Durchmesser von etwa 1 bis 200 µm, insbesondere etwa 5 bis 100 µm, und Dicken von etwa 0,1 bis 5 µm, insbesondere um etwa 0,5 µm. Ihre spezifische freie Oberfläche (BET) beträgt im allgemeinen 0,1 bis 5 m²/g.

Die erfindungsgemäß herzustellenden Glanzpigmente zeichnen sich durch eine Mehrfachbeschichtung des metallischen Substrats aus.

Die Schicht (A) enthält als wesentliche Bestandteile Aluminiumoxid, Aluminiumoxidhydrat und bevorzugt Siliciumoxid und Siliciumoxidhydrat sowie auch deren Mischungen.

Die Dicke der Schicht (A) beträgt im allgemeinen 20 bis 800 nm, bevorzugt 50 bis 600 nm. Da die Schicht (A) im wesentlichen den Farbton der Pigmente bestimmt, hat sie für ein besonders ausgeprägtes Farbenspiel zeigende und daher auch bevorzugte Glanzpigmente, die nur ein Schichtpaket (A) und (B) aufweisen, eine Mindestschichtdicke von etwa 100 nm.

Mit wachsender Schichtdicke von (A) durchläuft man bei den mit der Schicht (A) und der schwarzen Schicht (B) belegten Pigmenten bei einem Betrachtungswinkel von 25° mehrmals nacheinander die Interferenzfarben blau, grün, gold, rot. Die Winkelabhängigkeit des Farbtons nimmt von der ersten Interferenzfarbenserie nach höheren Serien (also dicker werdenden Schichten (A)) zu. So kippt beispielsweise ein rötlicher Goldton der ersten Serie winkelabhängig ab in ein grünliches Gold, während ein solcher Farbton aus der zweiten oder dritten Interferenzserie in die Komplementärfarbe, ein grünstichiges Blau, umschlägt.

Zum Aufbau der Schicht (B) sind vor allem die nichtselektiv absorbierenden Sulfide von Cobalt und Nickel, besonders von Eisen, Chrom und Wolfram und ganz besonders von Molybdän geeignet.

Die Metallsulfide können einzeln, aber auch in Form von Gemischen, z. B. MoS₂/WS₂, vorliegen. Weiterhin können neben den Sulfiden auch Oxide des jeweiligen Metalls, z. B. MoS₂ und niedere Molybdänoxide, und insbesondere auch die Metalle selbst, z. B. MoS₂ und Molybdän, anwesend sein.

Bedingt durch die erfindungsgemäße Art der Herstellung der Glanzpigmente, bei der die mit dem entsprechenden Metalloxid oder dem Metall selbst belegten Pigmente zur Bildung des Metallsulfids in einer schwefelhaltigen Atmosphäre erhitzt werden, ist bei unvollständiger Umsetzung in der Regel eine Anreicherung des Sulfids an der Oberfläche bzw. im äußeren Bereich der Schicht (B) zu beobachten, wobei der innen (näher zum Substrat) liegende Bereich der Schicht (B) nahezu kein Sulfid aufweisen und im wesentlichen nur aus dem jeweiligen Metalloxid bzw. dem jeweiligen Metall bestehen kann.

Die nichtselektiv absorbierende Schicht (B) soll selbstverständlich nicht deckend, sondern für Licht zumindest teilweise durchlässig (semitransparent) sein. Je nach den optischen Eigenschaften der Metallsulfide beträgt die Dicke der Schicht (B) daher üblicherweise 1 bis 100 nm. Bei absorbierenden, hochbrechenden Materialien wie Molydänsulfid sind in der Regel Schichtdicken von 5 bis 20 nm bevorzugt.

Sind mehrere (z.B. 2, 3 oder 4) Schichtpakete (A) + (B) enthalten, dann ist die Schicht (A) insbesondere 20 bis 400 nm dick und die Schicht (B) bevorzugt 2 bis 5 nm dick. Bevorzugt sind jedoch die Glanzpigmente mit nur einem Schichtpaket (A) + (B).

Die erfindungsgemäß herzustellenden Glanzpigmente können noch eine äußere Schicht (C) aufweisen, die aus farblosen oder selektiv absorbierenden Metalloxiden aufgebaut ist und zur Stabilisierung der Schicht (B) gegen Umwelteinflüsse beiträgt, wenn diese metallische oder niederoxidische Bestandteile enthält.

Für die Schicht (C) geeignete Metalloxide sind z. B. Aluminiumoxid, Aluminiumoxidhydrat, Zinnoxid, Titandioxid, Zirkonoxid, Eisen(III)oxid, Chrom(III)oxid und bevorzugt Siliciumoxid und - oxidhydrat.

Die Dicke der Schicht (C) beträgt im allgemeinen etwa 1 bis 400 nm, vorzugsweise 5 bis 250 nm.

Selbstverständlich kann auch die Schicht (C) zur Interferenz des Pigmentes beitragen und dabei die Interferenzenreihe an der durch das mit (A) und (B) beschichtete Substrat bestimmten Stelle fortsetzen. Dies ist beispielsweise der Fall, wenn Zirkon- oder Titanoxid als Schicht (C) aufgebracht werden. Besteht dagegen die Schicht (C) im wesentlichen aus Siliciumoxid, so wird sich diese Schicht im Anwendungsmedium (z.B. Lacken, Druckfarben oder Tinten), das einen ähnlichen Brechungsindex aufweist, koloristisch kaum bemerkbar machen.

Farbige Metalloxide wie Eisen- und Chromoxid werden die Interferenzfarbe des Mehrschichtsystems durch Beimischen ihrer Absorptionsfarbe modifizieren und mit zunehmender Schichtdicke schließlich überdecken.

Insgesamt zeichnen sich bei den erfindungsgemäß herzustellenden Glanzpigmenten alle Schichten durch ihren gleichmäßigen, homogenen und filmartigen Aufbau und ihre Fähigkeit zur Interferenz auch der dickeren Schichten aus, so daß kräftige Interferenzfarben zeigende Mehrschichtsysteme entstehen, bei denen die Substratteilchen an allen Seiten beschichtet sind.

Zudem zeigen die mit im wesentlichen aus Metallsulfid bestehenden Schichten (B) belegten Glanzpigmente hohe Beständigkeit gegenüber Chemikalien, z. B. Säuren und Basen, und Schwitzwasserbeständigkeit in Lacken.

Bei dem erfindungsgemäßen Verfahren zur Herstellung der Glanzpigmente werden die einzelnen Schichten durch Gasphasenzersetzung geeigneter flüchtiger Metallverbindungen (chemical vapor deposition, CVD) oder naßchemisch durch hydrolytische Zersetzung insbesondere organischer Metallverbindungen aufgebracht.

Selbstverständlich können beide Vorgehensweisen beliebig zur Herstellung der einzelnen Schichten kombiniert werden.

Zur Erzeugung der Silicium- und/oder Aluminiumoxidschichten (A) sind die naßchemische und die CVD-Verfahrensvariante gleichermaßen geeignet, jedoch wird meist die CVD-Variante vorzuziehen sein, da die metallsulfidhaltigen Schichten (B) erfindungsgemäß aus der Gasphase abgeschieden werden. Eine Zwischenisolierung und Trocknung des mit (A) belegten Pigments kann dann entfallen.

Bei der in der nicht vorveröffentlichten DE-A-44 05 492 beschriebenen naßchemischen Variante werden organische Silicium- und/oder Aluminiumverbindungen, bei denen die organischen Reste über Sauerstoffatome an die Metalle gebunden sind, in Gegenwart der Substratteilchen und eines organischen Lösungsmittels, in welchem die Metallverbindungen löslich sind, hydrolysiert.

Hierfür sind eine Vielzahl von organischen Lösungsmitteln geeignet, bevorzugt ist Isopropanol.

Bevorzugte Beispiele für die metallischen Ausgangsverbindungen sind die Acetylacetonate und insbesondere Alkoholate, vor allem C₁-C₄-Alkanolate, z.B. Aluminiumtriisopropanolat und Tetraethoxysilan.

Die Hydrolyse wird vorzugsweise in Gegenwart einer Base oder einer Säure als Katalysator durchgeführt. Hierfür eignen sich z.B. neben Alkalilaugen wie Natronlauge insbesondere wäßrige Ammoniaklösungen. Geeignete saure Katalysatoren sind beispielsweise Phosphorsäure und organische Säuren wie Essigsäure und Oxalsäure.

Wasser muß mindestens in der stöchiometrisch für die Hydrolyse erforderlichen Menge vorliegen, bevorzugt ist jedoch die 2 bis 100fache, insbesondere die 5 bis 20fache Menge.

Bezogen auf die eingesetzte Wassermenge, gibt man in der Regel 3 bis 40 Vol.-%, vorzugsweise 5 bis 30 Vol.-%, einer 25 gew.-%igen wäßrigen Ammoniaklösung zu.

Für die Temperaturführung hat es sich als vorteilhaft erwiesen, das Reaktionsgemisch innerhalb von 10 bis 48 h schrittweise auf Rückflußtemperatur zu erhitzen. Bei Verwendung von Isopropanol als Lösungsmittel rührt man das Gemisch zum Beispiel bevorzugt zunächst 4 bis 20 h bei 40°C, dann 4 bis 20 h bei 60°C und zum Schluß 2 bis 8 h bei 80°C.

Verfahrenstechnisch geht man bei Schritt a) des erfindungsgemäßen Herstellungsverfahrens zweckmäßigerweise wie folgt vor:

Man legt Substratteilchen, organisches Lösungsmittel, Wasser und Katalysator (Säure oder bevorzugt Base, insbesondere z.B. eine wäßrige Ammoniaklösung) vor und gibt die zu hydrolysierende Metallverbindung, pur oder gelöst, z.B. als 30 bis 70, bevorzugt 40 bis 60 vol.-%ige Lösung im organischen Lösungsmittel, zu. Erfolgt die Zugabe der Metallverbindung in einem Schritt, dann wird die Suspension anschließend wie oben beschrieben unter Rühren erhitzt. Man kann die Metallverbindung aber auch bei erhöhter Temperatur kontinuierlich zudosieren, wobei Wasser und Ammoniak vorgelegt oder ebenfalls kontinuierlich zudosiert werden können. Nach beendeter Beschichtung wird die Reaktionsmischung wieder auf Raumtemperatur abgekühlt.

Um eine Agglomeratbildung während des Beschichtungsvorgangs zu verhindern, kann die Suspension einer starken mechanischen Beanspruchung wie Pumpen, kräftigem Rühren oder Einwirken von Ultraschall unterzogen werden.

Gewünschtenfalls kann man den Beschichtungsschritt ein- oder mehrfach wiederholen.

Bei der in der nicht vorveröffentlichten DE-A-44 37 752 beschriebenen CVD-Variante werden Silane, die mindestens einen Alkanoyloxyrest enthalten, in der Gasphase mit Wasserdampf und gegebenenfalls Sauerstoff in Gegenwart bewegter Substratteilchen zersetzt.

Hierfür geeignete Silane entsprechen insbesondere der Formel

RₐSiX_{b}Y_{c}

in der die Variablen folgende Bedeutung haben:
- R: Alkyl, bevorzugt C₁-C₁₀-Alkyl, besonders bevorzugt C₁-C₆-Alkyl, das durch Chlor substituiert sein kann, ein- oder mehrfach ungesättigt sein kann und dessen Kohlenstoffkette durch eine oder mehrere Iminogruppen oder Sauerstoffatome in Etherfunktion unterbrochen sein kann; Phenyl, das durch C₁-C₂-Alkyl substituiert sein kann, oder Wasserstoff;
- X: Alkoxy, bevorzugt C₁-C₆-Alkoxy, besonders bevorzugt C₄-Alkoxy, vor allem tert.-Butoxy;
- Y: Alkanoyloxy, bevorzugt C₂-C₃-Alkanoyloxy, besonders bevorzugt Acetoxy;
- a: 0 bis 3, bevorzugt 0 bis 2, besonders bevorzugt 0;
- b: 0 bis 3, bevorzugt 1 bis 3, besonders bevorzugt 2;
- c: 1 bis 4, bevorzugt 1 bis 3, besonders bevorzugt 2,
wobei die Summe a+b+c=4 ist und die Reste R für a>1, die Reste X für b>1 und die Reste Y für c>1 jeweils gleich oder verschieden sein können.

Besonders geeignet sind die Silane, die bei Temperaturen ≤ 600°C, aus technischen Gründen insbesondere auch ≤ 300°C, einen ausreichend hohen Dampfdruck aufweisen, um eine einfache Verdampfung zu gewährleisten, und auch leicht mit Wasserdampf und/oder Luft zersetzt und als Oxid abgeschieden werden können. Selbstverständlich können auch Gemische verschiedener Silane eingesetzt werden.

Im einzelnen seien beispielhaft folgende bevorzugte Silane genannt:

Tetraacetoxysilan, Methoxy-, Ethoxy-, Propoxy-, Isopropoxy-, Butoxy-, Isobutoxy-, sec.-Butoxy- und tert.-Butoxytriacetoxysilan, Dimethoxy-, Diethoxy-, Dipropoxy-, Diisopropoxy-, Dibutoxy-, Diisobutoxy-, Di-sec.-butoxy- und Di-tert.-butoxydiacetoxysilan und Trimethoxy-, Triethoxy-, Tripropoxy-, Triisopropoxy-, Tributoxy-, Triisobutoxy-, Tri-sec.-butoxy-und Tri-tert.-butoxyacetoxysilan sowie auch Silane, die verschiedene Alkoxyreste enthalten, z.B. Methoxyethoxydiacetoxysilan.

Ganz besonders bevorzugt ist Di-tert.-butoxydiacetoxysilan.

Zur Durchführung der CVD-Variante empfiehlt sich wie allgemein für CVD-Verfahren die Verwendung eines Wirbelschichtreaktors, wie er beispielsweise in der EP-A 45 851 beschrieben ist. Die Substratteilchen werden im Reaktor unter Fluidisierung (Verwirbelung) mit einem inerten Wirbelgas wie Stickstoff auf die gewünschte Reaktionstemperatur (in der Regel 100 bis 600°C, bevorzugt 150 bis 300°C) erhitzt, Silan und Wasserdampf (sowie gegebenenfalls Sauerstoff) werden dann mit Hilfe inerter Trägergasströme (vorteilhaft Teilströmen des Wirbelgases) aus vorgeschalteten Verdampfergefäßen über getrennte Düsen eingeleitet.

Um homogene, die Substratteilchen vollständig umhüllende, filmartige Siliciumoxidschichten zu erhalten, wird die Silankonzentration zweckmäßigerweise bei ≤ 5 Vol.-%, vorzugsweise ≤ 2 Vol.-%, bezogen auf die Gesamtgasmenge im Reaktor, gehalten.

Die zur Zersetzung erforderliche Menge Wasserdampf hängt von der Konzentration des Silans ab und sollte mindestens der stöchiometrisch zur Hydrolyse erforderlichen Menge entsprechen, bevorzugt ist jedoch die 10 bis 100fache Menge.

Enthält das Silan Alkyl- oder Phenylsubstituenten R, so empfiehlt sich die Anwesenheit von Sauerstoff bei der Zersetzung, wenn Kohlenstoffreste, die sich in der Regel bei der alleinigen Verwendung von Wasserdampf bilden, in der abgeschiedenen Siliciumoxidschicht vermieden werden sollen.

Die metallsulfidhaltigen Schichten (B) werden beim erfindungsgemäßen Herstellungsverfahren vorteilhaft nach den beiden in der EP-A-579 091 beschriebenen Verfahrensvarianten auf die mit (A) beschichteten Substratteilchen aufgebracht, wobei entweder durch Gasphasenzersetzung flüchtiger Metallverbindungen in Gegenwart eines Inertgases oder von Sauerstoff und/oder Wasserdampf zunächst eine Metall- bzw. Metalloxidschicht abgeschieden wird, die dann durch Umsetzung mit einer flüchtigen schwefelhaltigen Verbindung oder mit Schwefeldampf in die gewünschte metallsulfidhaltige Schicht (B) überführt wird, oder die Schicht (B) direkt durch Gasphasenzersetzung flüchtiger Metallverbindungen in schwefelhaltiger Atmosphäre abgeschieden wird.

Neben den in der EP-A 579 091 genannten schwefelhaltigen organischen Verbindungen sind als bevorzugte Schwefellieferanten insbesondere Schwefelwasserstoff und vor allem Schwefel selbst zu nennen.

Beim Einsatz von elementarem Schwefel geht man verfahrenstechnisch zweckmäßigerweise so vor, daß man feingemahlenes Schwefelpulver zusammen mit dem Substratmaterial mischt und in den Reaktor gibt, etwa 1 bis 4 h inertisiert und anschließend unter Ausschluß von Sauerstoff auf die Reaktionstemperatur (im allgemeinen 200 bis 500°C, bevorzugt 300 bis 500°C, besonders bevorzugt 400 bis 450°C) erhitzt.

Die Umsetzung ist in der Regel in 1 bis 5 h, vorzugsweise 2 bis 3 h, beendet.

Eventuell vorhandene Schwefelreste lassen sich durch Sublimation im Inertgasstrom leicht entfernen. In der Regel wird dies jedoch nicht notwendig sein, da der Schwefel quantitativ (bis zu der für die Bildung des Metallsulfids stöchiometrisch erforderlichen Menge) umgesetzt wird und daher leicht in der dem gewünschten Sulfidgehalt der Schicht (B) entsprechenden Menge zugegeben werden kann. Bevorzugt wird so viel Schwefel eingesetzt, daß die metallische oder oxidische Ausgangsschicht zumindest von einer zusammenhängenden, dichten Sulfidschicht bedeckt ist.

Als Reaktor läßt sich für diese Umsetzung neben dem auch für die anderen CVD-Verfahrensschritte besonders geeigneten Wirbelschichtreaktor, wie er z. B. in der EP-A-45 851 beschrieben ist, vorteilhaft ein Einhalsrundkolben aus Quarzglas verwenden, der über einen Motor gedreht wird, mit Gaszu- und -ableitungen in der Drehachse versehen ist und von einem zweischaligen Klappofen beheizt wird ("Drehkugelofen").

Im Prinzip kann jeder beheizbare Mischer, der das Substratmaterial mittels entsprechender Einbauten schonend bewegt und eine Gaszu- und -ableitung gestattet, als Reaktor eingesetzt werden.

Für eine kontinuierliche Verfahrensführung im technischen Maßstab eignet sich z. B. auch ein Drehrohrofen, dem ein Gemisch von Substratteilchen und Schwefel (bzw. Substratteilchen und eine Schwefelwasserstoff/Inertgas-Mischung) fortlaufend zugeführt wird.

Die Metalloxidschicht (C) wird beim erfindungsgemäßen Herstellungsverfahren durch hinlänglich bekannte oxidative Gasphasenzersetzung der Metallcarbonyle (z. B. Eisenpentacarbonyl, Chromhexacarbonyl) bzw. hydrolytische Gasphasenzersetzung der Metallalkoholate oder Metallhalogenide (z. B. Titan- und Zirkon-tetran-und-isopropanolat, Titan- und Zirkontetrachlorid) (EP-A-33 457, EP-A-338 428) oder durch die oben beschriebene Gasphasenhydrolyse von Siliciumorganylen bzw. naßchemische hydrolytische Zersetzung von Silicium- oder Aluminiumalkoholaten aufgebracht.

Mit Hilfe des erfindungsgemäßen Herstellungsverfahrens können die mehrfach beschichteten Glanzpigmente in einfacher weise in großen Mengen reproduzierbar hergestellt werden. Es werden vollständig umhüllte Pigmentteilchen mit hoher Qualität der einzelnen Beschichtungen (homogen, filmartig) erhalten.

Die erfindungsgemäß hergestellten Glanzpigmente und Glanzpigmentmischungen eignen sich vorteilhaft für viele Zwecke, wie zur Einfärbung von Kunststoffen, Gläsern, keramischen Produkten, Zubereitungen der dekorativen Kosmetik und insbesondere von Lacken, insbesondere auch Automobillacken, Tinten und Druckfarben, vor allem Sicherheitsdruckfarben. Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäß hergestellten Pigmente auch vorteilhaft in Abmischung mit transparenten und deckenden Weiß-, Bunt- und Schwarzpigmenten sowie auch herkömmlichen transparenten, bunten und schwarzen Glanzpigmenten auf der Basis von metalloxidbeschichteten Glimmer- und Metallpigmenten, plättchenförmigen Eisenoxiden, Graphit, Molybdänsulfid und plättchenförmigen, organischen Pigmenten verwenden.

### Beispiele

### Herstellung und Anwendung von Glanzpigmenten

Bei der Einarbeitung der Pigmente in Lack wurden jeweils 0,4 g Pigment in 3,6 g eines Polyester-Mischlackes mit 21 Gew.-% Feststoffanteil eingerührt und 2 min im Red Devil dispergiert. Mit einer Rakel (160 µm Naßfilmdicke) wurden auf schwarzweißem Karton Abzüge der pigmentierten Lacke angefertigt.

Bei der Anwendung der Pigmente im Siebdruck wurden 10 g Pigment in 90 g einer handelsüblichen Bindemittellösung (22,5 g PVC"Mischpolymerisat Laroflex® MP45, 4,5 g Methoxypropylacetat, 13,5 g n-Hexyldiglykol, 49,5 g Butylglykol) eingerührt. Die so hergestellte Siebdruckfarbe wurde mit einer handelsüblichen Siebdruckmaschine (Siebmaschenweite 112 bis 150 µm) auf gestrichenes, titandioxidbeschichtetes Papier in einer Schichtdicke von 45 µm aufgebracht und an der Luft getrocknet.

### Beispiel 1

a) In einem mit Rückflußkühler und Rührapparatur versehenen Rundkolben wurden 100 g feinteiliges Aluminiumpulver (mittlerer Teilchendurchmesser 20 µm, BET-Oberfläche 4,5 m²/g) in 1,5 l Isopropanol aufgeschlämmt. Nach Zugabe von 400 ml Wasser und 40 ml einer 25 gew.-%igen wäßrigen Ammoniaklösung wurde die Suspension unter kräftigem Rühren auf 65°C erhitzt. Gleichzeitig wurde mit der Zudosierung eines Gemisches von 600 ml Isopropanol und 600 g Tetraethoxysilan begonnen (Dosiergeschwindigkeit 100 ml/h, 12 h). Nach einer Nachrührzeit von 10 h und Abkühlen der Suspension wurde das Produkt abfiltriert, gründlich mit Isopropanol gewaschen und bei 80°C getrocknet.
   Das beschichtete Aluminiumpulver hatte einen SiO₂-Gehalt von 59,3 Gew.-% und zeigte einen schwachen Grünstich.
b) Anschließend wurden 200 g des beschichteten Aluminiumpulvers im Wirbelschichtreaktor (beschrieben in der EP-A-571 836) unter Fluidisierung mit insgesamt 800 l/h Stickstoff auf 300°C erhitzt. Aus einer auf 60°C temperierten Verdampfervorlage wurden mit einem zusätzlichen Stickstoffstrom von 400 l/h 17 g Molybdänhexacarbonyl in 6 h in den Reaktor überführt und dort mit dem parallel über eine weitere Reaktoröffnung eingeleiteten Schwefelwasserstoff (5 l/h) zu sich filmartig abscheidendem Molybdänsulfid umgesetzt.
   Das erhaltene Pigment hatte einen Molybdängehalt von 2,9 Gew.-% und einen Schwefelgehalt von 1,73 Gew.-% und zeigte bei Applikation im Lack und im Siebdruck bei nahezu unverändert starkem metallischen Glanz eine kräftige, goldene Interferenzfarbe, die bei steileren Betrachtungswinkeln nach Grün abkippte. Eine mit einem kommerziellen Farbkopierer (Canon CLC 500) hergestellte Farbkopie des Siebdrucks zeigte kein Farbenspiel mehr, sondern lediglich eine Mischfarbe.

### Beispiel 2

a) Analog Beispiel 1a) wurden 100 g des Aluminiumpulvers unter Verwendung eines Gemisches von 580 ml Isopropanol und 580 g Tetraethoxysilan mit 57,3 Gew.-% SiO₂ (bezogen auf das Gewicht des beschichteten Pigmentes) beschichtet.
b) Anschließend wurden 156 g des SiO₂-beschichteten Aluminiumpulvers im Wirbelschichtreaktor unter Fluidisierung mit insgesamt 600 l/h Stickstoff auf 220°C erhitzt und analog Beispiel 1b) in etwa 8 h unter Verwendung von 24,4 g Mo(CO)₆ mit Molybdän belegt. Nach beendeter Molybdänabscheidung wurde den Wirbelgasen zur Passivierung der Molybdänoberfläche etwas Luft zugesetzt.
   Das Pigment hatte einen Molybdängehalt von 4,9 Gew.-% und zeigte eine kräftige, grüne Intereferenzfarbe, die bei steileren Betrachtungswinkeln nach Blau abkippte.
   75 g des Mo-beschichteten Pigments wurden dann in dem oben beschriebenen Drehkugelofen nach Inertisierung mit Stickstoff auf 400°C erhitzt und 2 h unter ständiger Bewegung mit einem Schwefelwasserstoffstrom von 10 l/h in Kontakt gebracht. Danach wurde erneut mit Stickstoff gespült und auf Raumtemperatur abgekühlt.

Das erhaltene Pigment hatte einen Schwefelgehalt von 0,44 Gew.-% und zeigte bei Applikation eine grünlich goldene Interferenzfarbe, die bei steileren Betrachtungswinkeln nach Blau abkippte.

### Beispiel 3

a) Eine Aufschlämmung von 100 g des Aluminiumpulvers aus Beispiel 1 in 1,5 l Isopropanol wurde unter kräftigem Rühren auf 65°C erhitzt und gleichzeitig mit parallel zudosierten Lösungen von 1) 400 ml Wasser, 40 ml einer 25 gew.-%igen wäßrigen Ammoniaklösung und 370 ml Isopropanol und 2) 600 g Tetraethoxysilan und 200 g Isopropanol versetzt (Dosiergeschwindigkeit für beide Lösungen 50 ml/h, 16 h). Nach einer Nachrührzeit von 8 h wurde analog Beispiel 1 a) aufgearbeitet.
   Das beschichtete Aluminiumpulver hatte einen SiO₂-Gehalt von 55,4 Gew.-% und zeigte einen schwachen Blaustich.
b) Analog Beispiel 2 b) wurden 245 g des SiO₂-beschichteten Aluminiumpulvers anschließend unter Fluidisierung mit 1200 l/h Stickstoff und Verwendung von 35,3 g Mo(CO)₆ mit Molybdän belegt.
   Das Pigment hatte einen Molybdängehalt von 5 Gew.-% und zeigte einen rotstichigen Blauton.
   100 g des Mo-beschichteten Pigments wurden dann mit 1 g feingemahlenem Schwefelpulver gemischt, im Drehkugelofen zunächst durch einstündiges Überleiten von 25 l/h Stickstoff inertisiert und anschließend unter einem Stickstoffstrom von 5 l/h in 30 min auf 400°C erhitzt. Nach 2 h wurde unter Stickstoff auf Raumtemperatur abgekühlt.

Das erhaltene Pigment hatte einen Schwefelgehalt von 0,55 Gew.-% und zeigte bei Applikation eine kräftige, rein blaue Interferenzfarbe, die bei steileren Betrachtungswinkeln in ein kräftiges Violett abkippte.

### Beispiel 4

Analog Beispiel 3a) und b) wurden 100 g Aluminiumpulver zunächst mit SiO₂ und Molybdän beschichtet und dann mit Schwefel umgesetzt, wobei jedoch 2 g feingemahlenes Schwefelpulver eingesetzt wurden.

Das erhaltene Pigment hatte einen Schwefelgehalt von 1,3 Gew.-% und zeigte bei Applikation eine grünstichig blaue Interferenzfarbe, die bei steileren Betrachungswinkeln in ein rotstichiges Blau abkippte.

## Patentansprüche

1. Verfahren zur Herstellung von goniochromatischen Glanzpigmenten auf der Basis von mehrfach beschichteten plättchenförmigen metallischen Substraten, die mindestens ein Schichtpaket aus
A) einer im wesentlichen aus Siliciumoxid, Siliciumoxidhydrat, Aluminiumoxid und/oder Aluminiumoxidhydrat bestehenden Schicht und
B) einer nichtselektiv absorbierenden, für sichtbares Licht zumindest teilweise durchlässigen, metallsulfidhaltigen Schicht
sowie gewünschtenfalls zusätzlich
C) eine äußere, im wesentlichen aus farblosem oder selektiv absorbierendem Metalloxid bestehende Schicht
aufweisen, dadurch gekennzeichnet, daß man
die Schichten (A) durch Gasphasenzersetzung flüchtiger Siliciumorganyle, die mindestens einen Alkanoyloxyrest enthalten, mit Wasserdampf und/oder Sauerstoff oder naßchemisch durch hydrolytische Zersetzung organischer Silicium- oder Aluminiumverbindungen, bei denen die organischen Reste über Sauerstoffatome an das Metall gebunden sind, in Gegenwart eines organischen Lösungsmittels, in welchem die Metallverbindungen löslich sind, und anschließende Trocknung,
die Schichten (B) durch Gasphasenzersetzung flüchtiger Metallverbindungen in Gegenwart eines Inertgases oder von Sauerstoff und/oder Wasserdampf und anschließende Umsetzung der gebildeten Metall- oder Metalloxidschicht mit einer flüchtigen schwefelhaltigen Verbindung oder Schwefeldampf oder direkt durch Gasphasenzersetzung der Metallverbindungen in Gegenwart einer flüchtigen schwefelhaltigen Verbindung oder Schwefeldampf,
die Schicht (C) durch Gasphasenzersetzung flüchtiger Metallverbindungen in Gegenwart von Sauerstoff und/oder Wasserdampf
auf die metallischen Substratplättchen aufbringt.

2. Verfahren nach Anspruch 1, dadurch gekennzeichnet, daß man als flüchtige Siliciumorganyle Silane der Formel
RₐSiX_{b}Y_{c}
einsetzt, in der die Variablen folgende Bedeutung haben:
Alkyl, bevorzugt C₁-C₁₀-Alkyl, besonders bevorzugt C₁-C₆-Alkyl, das durch Chlor substituiert sein kann, ein-oder mehrfach ungesättigt sein kann und dessen Kohlenstoffkette durch eine oder mehrere Iminogruppen oder Sauerstoffatome in Etherfunktion unterbrochen sein kann; Phenyl, das durch C₁-C₂-Alkyl substituiert sein kann, oder Wasserstoff;
X Alkoxy, bevorzugt C₁-C₆-Alkoxy, besonders bevorzugt C₄-Alkoxy, vor allem tert.-Butoxy;
Y Alkanoyloxy, bevorzugt C₂-C₃-Alkanoyloxy, besonders bevorzugt Acetoxy;
a 0 bis 3;
b 0 bis 3;
c 1 bis 4,
wobei die Summe a+b+c=4 ist und die Reste R für a>1, die Reste X für b>1 und die Reste Y für c>1 jeweils gleich oder verschieden sein können.

3. Verfahren nach Anspruch 1, dadurch gekennzeichnet, daß man als organische Silicium- oder Aluminiumverbindungen, bei denen die organischen Reste über Sauerstoffatome an das Metall gebunden sind, Silicium- oder Aluminium-C₁-C₄-alkanolate einsetzt.

## Claims

1. A process for producing goniochromatic luster pigments based on multiply coated platelet-shaped metallic substrates comprising at least one layer packet of
A) a layer consisting essentially of silicon oxide, silicon oxide hydrate, aluminum oxide and/or aluminum oxide hydrate, and
B) a nonselectively absorbing metal sulfide layer which is at least partially transparent to visible light,
and also, if desired, additionally
C) an outer layer which consists essentially of colorless or selectively absorbing metal oxide,
which comprises coating the metallic substrate platelets with
layers (A) by gas phase decomposition of volatile organosilicons that contain at least one alkanoyloxy radical using water vapor and/or oxygen or wet-chemically by hydrolytic decomposition of organic silicon or aluminum compounds in which the organic radicals are attached to the metal via oxygen atoms in the presence of an organic solvent in which the metal compounds are soluble and subsequent drying,
layers (B) by gas phase decomposition of volatile metal compounds in the presence of an inert gas or of oxygen and/or water vapor and subsequent reaction of the resulting metal or metal oxide layer with a volatile sulfur-containing compound or sulfur vapor or directly by gas phase decomposition of the metal compounds in the presence of a volatile sulfur-containing compound or sulfur vapor,
layer (C) by gas phase decomposition of volatile metal compounds in the presence of oxygen and/or water vapor.

2. A process as claimed in claim 1, wherein the volatile organosilicons used are silanes of the formula
RₐSiX_{b}Y_{c}
where
R is alkyl, preferably C₁-C₁₀-alkyl, particularly preferably C₁-C₆-alkyl, which can be substituted by chlorine, can be monounsaturated or polyunsaturated and whose carbon chain can be interrupted by one or more imino groups or oxygen atoms in an ether function; phenyl, which can be C₁-C₂-alkyl-substituted, or hydrogen;
X is alkoxy, preferably C₁-C₆-alkoxy, particularly preferably C₄-alkoxy, especially tert-butoxy;
Y is alkanoyloxy, preferably C₂-C₃-alkanoyloxy, particularly preferably acetoxy;
a is from 0 to 3;
b is from 0 to 3;
c is from 1 to 4,
the sum a+b+c is 4 and the radicals R for a>1, the radicals X for b>1 and the radicals Y for c>1 can each be identical or different.

3. A process as claimed in claim 1, wherein the organic silicon or aluminum compounds in which the organic radicals are attached to the metal via oxygen atoms are silicon or aluminum C₁-C₄-alkoxides.

## Revendications

1. Procédé de préparation de pigments brillants goniochromatiques à base de substrats métalliques sous forme de plaquettes à revêtements multiples, qui comprennent au moins un ensemble de couches formé par
A) une couche constituée essentiellement d'oxyde de silicium, d'oxyde de silicium hydraté, d'oxyde d'aluminium et/ou d'oxyde d'aluminium hydraté et
B) une couche contenant un sulfure métallique, à absorption non sélective, au moins partiellement transparente pour la lumière visible,
et éventuellement en plus
C) une couche externe constituée essentiellement d'un oxyde métallique incolore ou à absorption sélective,
caractérisé en ce que l'on applique sur les plaquettes de substrat métalliques,
les couches (A) au moyen d'une décomposition en phase gazeuse de composés organiques volatils du silicium, qui contiennent au moins un reste alcanoyloxy, avec de la vapeur d'eau et/ou de l'oxygène ou par voie chimique humide au moyen d'une décomposition par hydrolyse de composés organiques du silicium ou de l'aluminium, dans lesquels les résidus organiques sont liés au métal par des atomes d'oxygène, en présence d'un solvant organique, dans lequel les composés métalliques sont solubles, suivie par un séchage,
les couches (B) au moyen d'une décomposition en phase gazeuse de composés métalliques volatils en présence d'un gaz inerte ou d'oxygène et/ou de vapeur d'eau, suivie par une réaction de la couche métallique ou d'oxyde métallique formée avec un composé volatil contenant du soufre ou de la vapeur de soufre ou bien directement au moyen d'une décomposition en phase gazeuse des composés métalliques en présence d'un composé volatil contenant du soufre ou de la vapeur de soufre,
la couche (C) au moyen d'une décomposition en phase gazeuse de composés métalliques volatils en présence d'oxygène et/ou de vapeur d'eau.

2. Procédé selon la revendication 1, caractérisé en ce que l'on met en oeuvre en tant que composés organiques du silicium volatils, des silanes de formule
RₐSᵢX_{b}Y_{c}
dans laquelle
R représente un groupe alkyle, de préférence alkyle en C₁ à C₁₀, de manière particulièrement préférée alkyle en C₁ à C₆, qui peut être substitué par du chlore, peut être insaturé une ou plusieurs fois et dont la chaîne carbonée peut être interrompue par un ou plusieurs groupes imino ou atomes d'oxygène en liaison éther;
un groupe phényle, qui peut être substitué par un groupe alkyle en C₁ à C₂, ou un atome d'hydrogène;
X représente un groupe alcoxy, de préférence alcoxy en C₁ à C₆, de manière particulièrement préférée alcoxy en C₄, en particulier tert-butoxy;
Y représente un groupe alcanoyloxy, de préférence alcanoyloxy en C₂ à C₃, de manière particulièrement préférée acétoxy;
a vaut de 0 à 3;
b vaut de 0 à 3;
c vaut de 1 à 4;
où la somme a+b+c=4 et les résidus R pour a>1, les résidus X pour b>1 et les résidus Y pour c>1 peuvent être respectivement identiques ou différents.

3. Procédé selon la revendication 1, caractérisé en ce que l'on met en oeuvre des alcanolates en C₁ à C₄ d'aluminium ou de silicium, en tant que composés organiques du silicium ou de l'aluminium, dans lesquels les résidus organiques sont liés au métal par des atomes d'oxygène.
